# DEMANDE DE BREVET EUROPEEN

(11) **EP 0 967 268 A1**
(43) Date de publication de la demande: **29.12.1999**
(21) Numéro de dépôt: 99401525.3
(22) Date de dépôt: 18.06.1999
(51) Int. Cl.: C12M 1/00, B01L 7/02, G05D 27/02

(54) **Enceinte de travail thermostatée**

(30) Priorité: 24.06.1998 FR 9808013
(71) Demandeur: Jouan, 44800 Saint Herblain (FR)
(72) Inventeur: Marchal, Norbert, 49300 Cholet (FR)
(74) Mandataire: Blot, Philippe Robert Emile

(57) **Abrégé**

L'enceinte de travail thermostatée comporte une chambre de travail (10) délimitant un espace de confinement (17) propre à la mise en oeuvre d'un travail, des moyens (68A, 70A, 72A) de maintien d'une température régulée dans une zone thermostatée principale (66A) définie autour de la chambre de travail (10), et une boucle de dérivation (14) dont les deux extrémités (28, 30) débouchent dans l'espace de confinement (17), laquelle boucle de dérivation (14) comporte au moins un capteur (34, 36, 38, 40) de mesure d'une variable caractéristique de l'atmosphère régnant dans ledit espace de confinement (17). Chaque capteur (34, 36, 38, 40) est disposé dans une zone thermostatée secondaire (66B) définie autour d'un tronçon de la boucle de dérivation (14) comportant le ou chaque capteur (34, 36, 38, 40). Elle comporte des moyens (68B, 70B, 728) de maintien de la zone thermostatée secondaire (66B) à une température égale à la température de la zone thermostatée principale (25 ; 66A).

Application aux incubateurs à dioxyde de carbone.

## Description

La présente invention concerne une enceinte de travail thermostatée du type type comportant une chambre de travail délimitant un espace de confinement propre à la mise en oeuvre d'un travail, des moyens de maintien d'une température régulée dans une zone thermostatée principale définie autour de la chambre de travail, et une boucle de dérivation pour la circulation d'une partie de l'atmosphère régnant dans ledit espace de confinement, les deux extrémités de la boucle de dérivation débouchant dans l'espace de confinement, laquelle boucle de dérivation comporte au moins un capteur de mesure d'une variable caractéristique de l'atmosphère régnant dans ledit espace de confinement.

Une telle enceinte de travail thermostatée constitue par exemple un incubateur à dioxyde de carbone. Ce dernier est couramment utilisé pour l'incubation de cellules humaines ou encore d'embryons.

Dans les incubateurs à dioxyde de carbone connus, les moyens de maintien de la température régulée dans la zone thermostatée comportent un fluide caloporteur dans lequel baigne la chambre de travail. Ce fluide caloporteur est de l'eau ou de l'air, chauffé pour être maintenu à une température constante et notamment égale à 37°C.

La teneur en dioxyde de carbone de l'atmosphère à l'intérieur de l'espace de confinement est maintenue par injection de dioxyde de carbone sensiblement égale à 5 %. De même, la teneur en eau de l'atmosphère est généralement régulée pour que celle-ci soit sensiblement égale à 97 %. La teneur en oxygène peut également être contrôlée.

Afin d'assurer une régulation satisfaisante de la température, de la teneur en dioxyde de carbone, de l'hygrométrie ou encore d'autres valeurs caractéristiques de l'atmosphère, plusieurs capteurs sont mis en contact avec l'atmosphère régnant dans l'espace de confinement.

Du fait des expériences pratiquées dans l'espace de confinement, celui-ci peut être contaminé. Ainsi, si les capteurs sont implantés directement dans la chambre de travail, il convient de nettoyer et de décontaminer ceux-ci, ce qui constitue une opération délicate.

De plus, les caractéristiques de l'atmosphère dans l'espace de confinement sont modifiées par la chaleur dégagée par les capteurs.

Afin de remédier à ces problèmes, il est connu de prévoir une boucle de dérivation dans laquelle circule une partie de l'atmosphère contenue dans l'espace de confinement. Cette boucle est piquée à ses deux extrémités sur la chambre de travail.

Les capteurs devant être mis en oeuvre, à l'exception des sondes de température, sont disposés successivement le long de la boucle de dérivation.

Cette solution permet de séparer les capteurs de la chambre de travail, remédiant ainsi à certains des inconvénients mentionnés précédemment.

Toutefois, la boucle de dérivation portant les capteurs chemine hors de la zone thermostatée. Ainsi, les capteurs effectuent leur mesure à la température ambiante du local dans lequel l'enceinte de travail thermostatée est installée.

Du fait que la température ambiante est généralement inférieure à la température imposée dans l'espace de confinement, il existe des risques de condensation dans les capteurs, le taux d'humidité de l'atmosphère étant élevé.

Pour prévenir une telle condensation, il est nécessaire de prévoir, en amont des capteurs, des moyens de réduction du taux d'humidité, par exemple par déshumidification ou chauffage de l'atmosphère.

Quels que soient les moyens utilisés pour prévenir la condensation, ceux-ci peuvent provoquer un vieillissement prématuré des capteurs ou engendrer des mesures erronées.

De plus, afin d'analyser le taux d'humidité, il convient de connaître la température dans l'espace de confinement et la température à l'entrée des capteurs. Ainsi, le calcul du taux d'humidité nécessite des moyens complexes et notamment la présence d'un capteur de température dans l'espace de travail.

L'invention a pour but d'apporter une solution aux problèmes mentionnés précédemment, en permettant le déport des capteurs sur une boucle de dérivation qui est piquée sur la chambre de travail et dans laquelle circule une partie de l'atmosphère de l'espace de confinement, sans nécessiter la mise en oeuvre de moyens de réduction du taux d'humidité, cette solution étant peu sensible aux différences de température avec le milieu ambiant. A cet effet, l'invention a pour objet une enceinte de travail thermostatée, du type précité, caractérisée en ce que le ou chaque capteur est disposé dans une zone thermostatée secondaire définie autour d'au moins un tronçon de la boucle de dérivation comportant le ou chaque capteur, et en ce qu'elle comporte des moyens de maintien de la zone thermostatée secondaire à une température égale à la température de la zone thermostatée principale.

Suivant des modes particuliers de réalisation, l'enceinte de travail thermostatée comporte l'une ou plusieurs des caractéristiques suivantes :
- les zones thermostatées principale et secondaire sont en communication et forment une zone thermostatée unique, et les moyens de maintien d'une température régulée de la zone thermostatée unique comporte une unité unique de régulation de la température dans la zone thermostatée unique ;
- les zones thermostatées principale et secondaire sont indépendantes, et sont chacune associées à des moyens propres de maintien de ladite température régulée commune ;
- ladite boucle de dérivation comporte une chambre de mesure traversée par une partie de l'atmosphère régnant dans l'espace de confinement, le ou chaque capteur ayant une partie active de mesure adaptée pour travailler dans ladite chambre de mesure ;
- elle comporte au moins deux capteurs, et ladite boucle de dérivation comporte, pour chaque capteur, une chambre de mesure distincte traversée par une partie de l'atmosphère régnant dans ledit espace de confinement, chaque capteur ayant une partie active de mesure adaptée pour travailler dans la chambre de mesure propre au capteur considéré ;
- ladite boucle de dérivation comporte, en entrée, un filtre d'arrêt d'au moins certains contaminants présent dans l'espace de confinement ;
- ladite boucle de dérivation comporte une pompe de mise en circulation d'une partie de l'atmosphère, suivant un trajet défini par ladite boucle de dérivation, et ladite pompe est disposée en dehors de l'une et l'autre des zones thermostatées principale et secondaire ;
- la zone thermostatée secondaire est munie de moyens d'accès direct aux capteurs depuis le milieu ambiant ; et
- elle comporte des moyens de régulation d'au moins une variable caractéristique de l'atmosphère de l'espace de confinement, à partir d'au moins une valeur caractéristique relevée par au moins un capteur.

L'invention sera mieux comprise à la lecture de la description qui va suivre, donnée uniquement à titre d'exemple et faite en se référant aux dessins, sur lesquels :
La figure 1 est une vue schématique d'un premier mode de réalisation d'une enceinte de travail thermostatée selon l'invention ; et
Les figures 2 et 3 sont des vues schématiques de variantes de réalisation de l'enceinte de travail thermostatée de la figure 1.

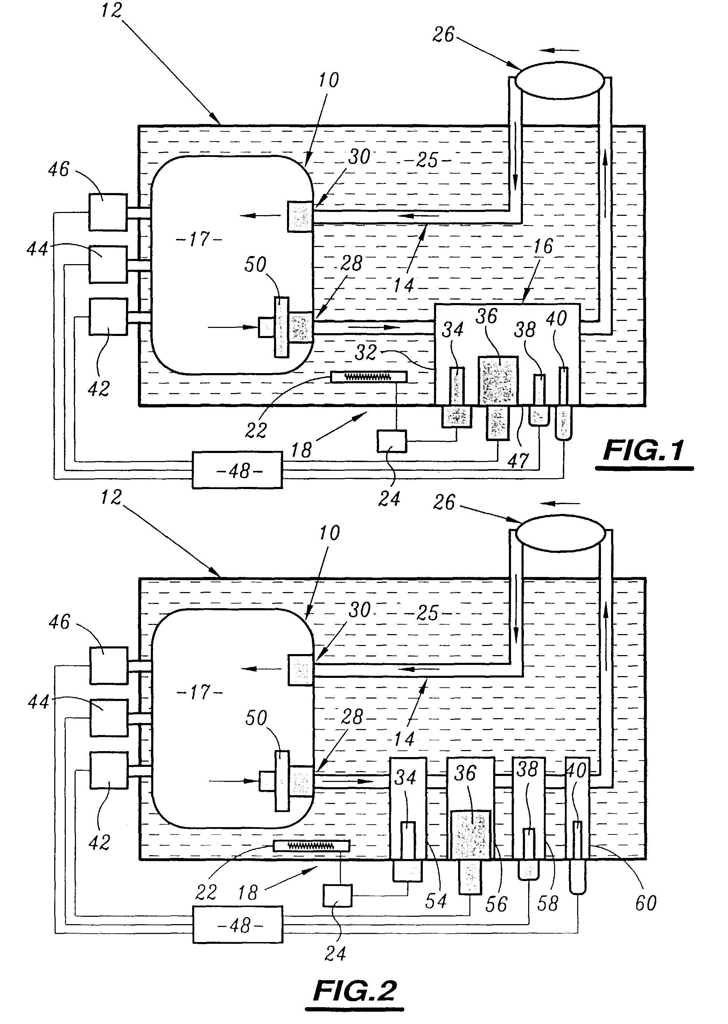

L'enceinte de travail thermostatée représentée sur la figure 1 comporte une chambre de travail 10 disposée complètement dans une cuve thermostatée 12. Une boucle de dérivation 14 est piquée sur la cuve thermostatée 12. La boucle comporte un ensemble 16 de capteurs adaptés pour la mesure de variables caractéristiques de l'atmosphère circulant dans celle-ci.

La chambre de travail 10 est constituée d'un caisson muni d'une ouverture d'accès. Elle délimite un espace de confinement 17 propre à la mise en oeuvre d'un travail, tel que l'incubation de cellules ou d'embryons humains.

La chambre de travail 10 est totalement baignée à l'intérieur d'un fluide caloporteur, tel que de l'eau, contenu à l'intérieur de la cuve thermostatée 12.

La chambre 10 est associée à des moyens 18 de régulation de la température du fluide caloporteur. Ces moyens sont constitués par exemple de résistances chauffantes 22 installées dans la cuve thermostatée 12. Les résistances chauffantes sont pilotées par un régulateur de température 24.

La cuve thermostatée 12 assure le maintien d'une température régulée, par exemple constamment égale à 37°C, dans toute la zone emplie du fluide caloporteur. Cette zone définit une zone thermostatée 25 autour de la chambre de travail 10.

La boucle de dérivation 14 débouche à ses deux extrémités dans la chambre de travail 10. Elle chemine partiellement au travers de la zone thermostatée 25 et partiellement au dehors de celle-ci, c'est-à-dire dans le milieu ambiant dans lequel est installée l'enceinte de travail.

La boucle de dérivation 14 comporte, sur un tronçon disposé hors de la zone thermostatée 20, une pompe 26 pour la mise en circulation d'une partie de l'atmosphère prélevée dans l'espace de confinement. L'atmosphère prélevée chemine alors dans la boucle de dérivation 14 depuis une entrée 28 de la dérivation jusqu'à une sortie 30.

En aval de l'entrée 28 est prévue, sur la boucle de dérivation 14, une chambre de mesure 32 sur laquelle est monté l'ensemble 16 de capteurs. La chambre de mesure 32 est disjointe et distincte de la chambre de travail 10. Elle est liée à celle-ci par des tronçons de liaison de la boucle de dérivation. Les tronçons de liaison sont formés par des conduites de section réduite par rapport aux sections des chambres 10 et 32. Chacun des capteurs a sa partie active de mesure adaptée pour travailler dans la chambre de mesure 32.

Dans le mode de réalisation représenté, quatre capteurs sont implantés dans la chambre de mesure 32.

Un premier capteur 34 constitue une sonde de température. ll est relié au régulateur 24 pour la commande des résistances chauffantes 22.

Les trois autres capteurs 36, 38, 40 sont adaptés respectivement pour la mesure de teneur en eau, en dioxyde de carbone et en oxygène de l'atmosphère présente dans la chambre de mesure 32.

Selon l'invention, la chambre de mesure 32 est disposée dans la zone thermostatée 25, de sorte que les capteurs 34, 36, 38 et 40 effectuent des mesures des variables caractéristiques de l'atmosphère dans des conditions de température analogues à celles régnant dans de l'espace de confinement 17.

Avantageusement, la chambre de mesure 32 comporte des moyens d'accès à l'ensemble 16 de capteurs, directement depuis le milieu ambiant, sans qu'il soit nécessaire de pénétrer dans la cuve 12 après l'avoir vidangée. Ces moyens d'accès comportent par exemple un panneau amovible 47 portant les capteurs et formant une paroi isolant l'intérieur de la chambre de mesure 32 du milieu ambiant.

Par ailleurs, l'enceinte de travail 10 comporte trois modules 42, 44, 46 de commande de la teneur respectivement en eau, en dioxyde de carbone et en oxygène de l'atmosphère dans l'espace de confinement 25.

Ces modules de commande 42, 44, 46 sont reliés à une unité centrale de pilotage 48 elle-même reliée aux capteurs 36, 38, 40 de mesure des teneurs en eau, en dioxyde de carbone et en oxygène.

L'unité centrale de pilotage 48 est adaptée pour piloter les modules de commande 42, 44, 46 afin que ceux-ci injectent ou prélèvent de l'eau, du dioxyde de carbone ou de l'oxygène dans l'espace de confinement 17.

Ces trois modules sont disposés au dehors de la zone thermostatée 25 et sont reliés à la chambre de travail 10 par des conduites traversant la cuve thermostatée 12.

Enfin, l'entrée 28 de la boucle de dérivation est avantageusement équipée d'un filtre 50 adapté pour l'élimination de contaminants éventuels présents dans la chambre de travail 10. Ce filtre est par exemple de type HEPA (haute efficacité particules air) comportant un tamis dont la taille des mailles est égale à 0,2 µm. Ce filtre est adapté pour protéger l'ensemble de capteurs disposé en aval sur la boucle de dérivation 14 vis-à-vis d'éventuels contaminants rencontrés dans l'espace de confinement 17.

Dans le cas de contaminants radioactifs, le filtre 50 comporte des charbons actifs.

L'enceinte de travail thermostatée fonctionne de la manière suivante.

Pendant toute l'utilisation de l'enceinte, les moyens 18 de maintien de la température régulée assurent que la température dans la zone thermostatée 25 soit maintenue à la température de consigne.

A cet effet, le régulateur 24 commande les résistances chauffantes 22 en fonction des valeurs relevées par la sonde de température 34.

La pompe 26 assure la circulation permanente d'une partie de l'atmosphère de l'espace de confinement au travers de la boucle de dérivation 14. Ainsi, l'atmosphère prélevée circule au travers de la chambre de mesure 32 où elle est mise en contact avec l'ensemble 16 des capteurs. La chambre étant disposée dans la zone thermostatée 25, l'atmosphère qui y règne est à une température analogue à la température de l'espace de confinement 17.

Dans ces conditions, les valeurs de température relevées par la sonde de température 34 correspondent exactement aux valeurs de température qui seraient relevées par une sonde placée directement dans la chambre de travail 10.

De même, les valeurs caractéristiques relevées par les capteurs 36, 38 et 40 concernant la teneur en différents gaz de l'atmosphère de la chambre de mesure 32 correspondent exactement aux teneurs de l'atmosphère dans l'espace de confinement, puisque la température dans la chambre de mesure et dans la chambre de travail 10 sont identiques.

A partir des valeurs caractéristiques relevées par les capteurs 36, 38 et 40, l'unité de pilotage 48 assure le pilotage des modules 42, 44, 46 pour garantir des teneurs contrôlées, en oxygène, en dioxyde de carbone et en eau de l'atmosphère contenue dans la chambre de travail 10.

On comprend qu'avec un tel agencement, les mesures étant effectuées alors que l'atmosphère est maintenue à la même température que celle de la chambre de travail, ces mesures sont fiables.

De plus, les capteurs peuvent fonctionner de manière satisfaisante sans qu'il soit nécessaire de modifier la composition de l'atmosphère avec laquelle ils sont mis en contact.

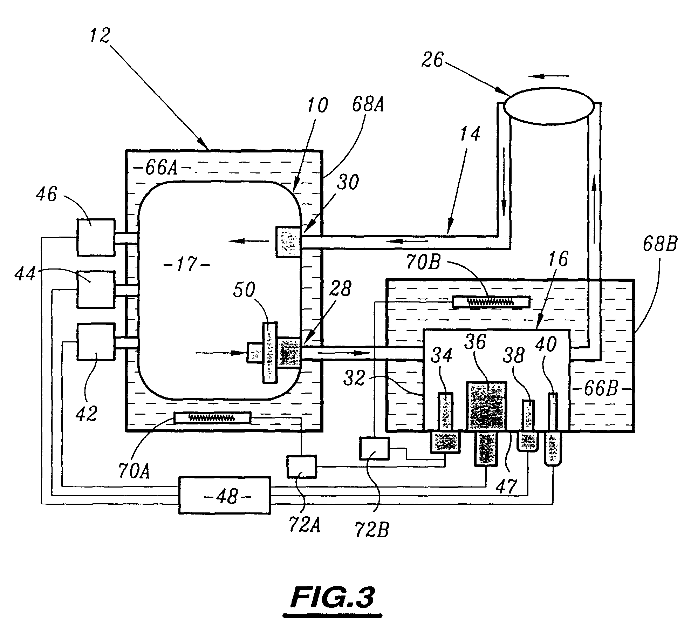

Sur les figures 2 et 3, sont représentées des variantes de réalisation de l'enceinte de travail thermostatée de la figure 1.

Sur ces figures, les éléments identiques ou analogues sont désignés par les mêmes numéros de référence que sur la figure 1.

Dans le mode de réalisation de la figure 2, l'enceinte de travail thermostatée ne diffère qu'en ce que la chambre de mesure unique 32 de la figure 1 est remplacée par quatre chambres de mesure distinctes, notées 54, 56, 58, 60, dans chacune desquelles est disposé un capteur unique.

Les chambres 54, 56, 58, 60 sont disposées en série sur la boucle de dérivation 14 en aval de l'entrée 28. Chaque chambre est baignée par le fluide caloporteur, de sorte que les chambres sont toutes disposées dans la zone thermostatée 25 assurant ainsi un travail des capteurs 34, 36, 38, 40 à une température identique à celle de l'espace de confinement 17.

Chaque chambre de mesure 54 à 60 comporte des moyens propres d'accès direct au capteur qui y est implanté depuis le milieu ambiant.

Dans le mode de réalisation de la figure 3, la chambre de travail 10 et la chambre de mesure 12 sont disposées dans deux zones thermostatées distinctes et indépendantes.

Ainsi, l'enceinte de travail 10 est disposée dans une zone thermostatée principale 66A alors que la chambre de mesure 32 est disposée dans une enceinte thermostatée secondaire 66B.

Les zones thermostatées principale et secondaire 66A, 66B sont définies chacune par une cuve thermostée 68A, 68B associée à des résistances électriques 70A, 70B alimentées chacune par une unité de régulation 72A, 72B. Les deux unités de régulation 72A, 72B sont reliées à la sonde de température 34.

Les deux unités de régulation 72A, 72B sont adaptées pour imposer des températures rigoureusement identiques dans la zone thermostatée principale 66A et dans la zone thermostatée secondaire 66B.

Les tronçons de la boucle de dérivation 14 immédiatement en aval de l'entrée 28 et immédiatement en amont de la sortie 30 traversent le milieu ambiant pour rejoindre les deux zones thermostatées 66A, 66B. Sur ces tronçons de liaison dans le milieu ambiant, la boucle de dérivation est recouverte d'un revêtement réalisé dans un matériau calorifuge.

Comme dans les modes de réalisation précédents, les capteurs effectuent des mesures sur l'atmosphère circulant dans la boucle de dérivation 14, alors que celle-ci est rigoureusement à la même température que l'atmosphère de l'espace de confinement 17.

Les moyens de maintien de la température comportant, dans les exemples décrits, de l'eau chauffée par des résistances électriques, comportent, en variante, des résistances chauffantes appliquées directement sur la chambre de travail. Ils peuvent également comporter un solide caloporteur dans lequel est intégrée la chambre de travail, des résistances chauffantes étant associées à ce solide.

## Revendications

1. Enceinte de travail thermostatée, du type comportant une chambre de travail (10) délimitant un espace de confinement (17) propre à la mise en oeuvre d'un travail, des moyens (12, 18 ; 68A, 70A, 72A) de maintien d'une température régulée dans une zone thermostatée principale (25 ; 66A) définie autour de la chambre de travail (10), et une boucle de dérivation (14) pour la circulation d'une partie de l'atmosphère régnant dans ledit espace de confinement (17), les deux extrémités (28, 30) de la boucle de dérivation (14) débouchant dans l'espace de confinement (17), laquelle boucle de dérivation (14) comporte au moins un capteur (34, 36, 38, 40) de mesure d'une variable caractéristique de l'atmosphère régnant dans ledit espace de confinement (17), caractérisée en ce que le ou chaque capteur (34, 36, 38, 40) est disposé dans une zone thermostatée secondaire (25; 66B) définie autour d'au moins un tronçon de la boucle de dérivation (14) comportant le ou chaque capteur (34, 36, 38, 40), et en ce qu'elle comporte des moyens (12, 18; 68B, 70B, 72B) de maintien de la zone thermostatée secondaire (25; 66B) à une température égale à la température de la zone thermostatée principale (25 ; 66A).

2. Enceinte de travail thermostatée selon la revendication 1, caractérisée en ce que les zones thermostatées principale (25) et secondaire (25) sont en communication et forment une zone thermostatée unique, et les moyens de maintien d'une température régulée de la zone thermostatée unique (25) comporte une unité unique (18) de régulation de la température dans la zone thermostatée unique.

3. Enceinte de travail selon la revendication 1, caractérisée en ce que les zones thermostatées principale (66A) et secondaire (66B) sont indépendantes, et sont chacune associées à des moyens propres (68A, 70A, 72A ; 68B, 70B, 72B) de maintien de ladite température régulée commune.

4. Enceinte de travail thermostatée selon l'une quelconque des revendications précédentes, caractérisée en ce que ladite boucle de dérivation (14) comporte au moins une chambre de mesure (32 ; 54, 56, 58, 60) traversée par une partie de l'atmosphère régnant dans l'espace de confinement (17), le ou chaque capteur (34, 36, 38, 40) ayant une partie active de mesure adaptée pour travailler dans ladite chambre de mesure (32, 54, 56, 58, 60), et en ce que la ou chaque chambre de mesure (32, 54, 56, 58, 60) est disjointe de la chambre de travail (10), la chambre de travail (10) étant reliée à la ou chaque chambre de mesure par des tronçons de liaison de la boucle de dérivation.

5. Enceinte de travail thermostatée selon l'une quelconque des revendications 1 à 4, caractérisée en ce qu'elle comporte au moins deux capteurs (34, 36, 38, 40), et ladite boucle de dérivation (14) comporte, pour chaque capteur, une chambre de mesure distincte (54, 56, 58, 60) traversée par une partie de l'atmosphère régnant dans ledit espace de confinement (17), chaque capteur (34, 36, 38, 40) ayant une partie active de mesure adaptée pour travailler dans la chambre de mesure (54, 56, 58, 60) propre au capteur considéré.

6. Enceinte de travail thermostatée selon l'une quelconque des revendications précédentes, caractérisée en ce que ladite boucle de dérivation (14) comporte, en entrée (28), un filtre (50) d'arrêt d'au moins certains contaminants présent dans l'espace de confinement (17).

7. Enceinte de travail thermostatée selon l'une quelconque des revendications précédentes, caractérisée en ce que ladite boucle de dérivation (14) comporte une pompe (26) de mise en circulation d'une partie de l'atmosphère, suivant un trajet défini par ladite boucle de dérivation (14), et en ce que ladite pompe (26) est disposée en dehors de l'une et l'autre des zones thermostatées principale (25 ; 66A) et secondaire (25 ; 66B).

8. Enceinte de travail thermostatée selon l'une quelconque des revendications précédentes, caractérisée en ce que la zone thermostatée secondaire (25; 66B) est munie de moyens (47) d'accès direct aux capteurs (34, 36, 38, 40) depuis le milieu ambiant.

9. Enceinte de travail thermostatée selon l'une quelconque des revendications précédentes, caractérisée en ce qu'elle comporte des moyens (42, 44, 46, 48) de régulation d'au moins une variable caractéristique de l'atmosphère de l'espace de confinement (17), à partir d'au moins une valeur caractéristique relevée par au moins un capteur (34, 36, 38, 40).
